# EUROPEAN PATENT SPECIFICATION

(11) **EP 4 465 955 B1**
(45) Date of publication and mention of the grant of the patent: **30.07.2025**
(21) Application number: 22817573.3
(22) Date of filing: 10.11.2022
(51) Int. Cl.: A61K 8/27, A61K 8/21, A61K 8/362, A61K 8/44, A61Q 11/00

(54) **ORAL CARE COMPOSITION**
MUNDPFLEGEZUSAMMENSETZUNG
COMPOSITION DE SOINS BUCCO-DENTAIRES

(30) Priority: 19.01.2022 EP 22152330; 28.06.2022 EP 22181691
(43) Date of publication of application: 27.11.2024
(73) Proprietor: Unilever IP Holdings B.V., 3013 AL Rotterdam (NL); Unilever Global IP Limited, Wirral, Merseyside CH62 4ZD (GB)
(72) Inventor: ADAMS, Suzanne Elizabeth, 6708 WH Wageningen (NL); HUNT, Joanne Elizabeth, 6708 WH Wageningen (NL); SLOMKA, Vera, 6708 WH Wageningen (NL)
(74) Representative: Tansley, Sally Elizabeth
(86) International application number: PCT/EP2022/081429
(87) International publication number: WO 2023/138811

(56) References cited:
- WO-A1-2021/137154
- CN-A- 104 877 354
- CN-A- 110 105 772
- US-A- 4 256 731

## Description

### Field of the Invention

The present invention relates to an oral care tooth paste composition according to the appended claims and its use.

### Background of the Invention

The oral cavity contains many different species of bacteria. Some species of oral pathogenic bacteria have been implicated in the development of periodontal disease and tooth decay however it is believed that certain species of oral bacteria are beneficial for maintaining oral health. Without being bound by any theory, it is believed that lysine degradation by oral bacteria contributes to gingival deterioration and that biosynthesis of lysine by the oral microbiome can positively contributes to the promotion of gum health.

Prebiotic oral care compositions are disclosed in WO 2021/137154 A1 (3M INNOVATIVE PROPERTIES CO [US]) 8 July 2021 (2021-07-08), WO15099752, WO15099753 and WO 15099754.

It is believed that selective stimulation of beneficial oral bacteria to produce lysine may provide a valid preventative approach for oral health, for example in the prevention of periodontitis and gingivitis.

There remains a need for a composition that stimulates beneficial oral bacteria to produce lysine and thus aid the prevention of periodontitis.

### Summary of the Invention

The present invention relates to an oral care tooth paste composition as defined in the claims.

The invention further relates to an oral care tooth paste composition comprising particulate abrasive, aspartic acid or salt thereof and fumaric acid or salt thereof for use in preventing one or more of periodontitis, gingivitis, periodontitis, peri-implantitis, peri-implant mucositis, necrotizing gingivitis, gingival deterioration and detachment and mixtures thereof.

### Detailed Description of the Invention

Except in the examples, or where otherwise explicitly indicated, all numbers in this description indicating amounts of material or conditions of reaction, physical properties of materials and/or use may optionally be understood as modified by the word "about".

All amounts are by weight of the composition, unless otherwise specified.

It should be noted that in specifying any ranges of values, any upper value can be associated with any particular lower value.

Where a feature is disclosed with respect to a particular aspect of the invention (for example a composition of the invention), such disclosure is also to be considered to apply to any other aspect of the invention (for example a method of the invention) *mutatis mutandis* Any ingredients mentioned in this application that are natural or naturally derived have been sourced from Europe. Any reference to an oral care composition other than an oral care tooth paste composition is to be interpreted to be limited to an oral care tooth paste composition.

for the treatment of periodontitis, gingivitis, periodontitis, peri-implantitis, peri-implant mucositis, necrotizing gingivitis, gingival deterioration and detachment and mixtures thereof.

It is preferable if the aspartic acid or salt thereof is sodium aspartate. Preferably the level of aspartic acid or salt thereof is from 0.0001 to 5 wt% of the total composition, more preferably from 0.001 to 3 wt%. most preferably from 0.01 to 1 wt%.

It is preferable if the fumaric acid or salt thereof is sodium fumarate Preferably the level of fumaric acid or salt thereof is from 0.0001 to 5 wt% of the total composition, preferably from 0.001 to 3 wt%. most preferably from 0.01 to 1 wt%.

In a preferred embodiment of the invention the weight ratio of aspartic acid or salt thereof to fumaric acid or salt thereof is from 1:2 to 2.

The composition according to the invention may comprise an anti -microbial agents, preferred anti-microbial agents are a source of fluoride in which the source of fluoride is preferably selected from the group consisting of sodium fluoride, stannous fluoride, sodium monofluorophosphate, zinc ammonium fluoride, tin ammonium fluoride, calcium fluoride, cobalt ammonium fluoride and mixtures thereof.

In a further aspect of the invention preferred anti-microbial agents are water-soluble or sparingly water-soluble sources of metal salts. Preferred are zinc ions such as zinc chloride, zinc acetate, zinc gluconate, zinc sulphate, zinc fluoride, zinc citrate, zinc lactate, zinc oxide, zinc monoglycerolate, zinc tartrate, zinc pyrophosphate and zinc maleate; also preferred are stannous ions such as stannous fluoride and stannous chloride.

Further suitable anti-microbial agents include ethyl lauroyl arginate HCl, chlorhexidine, sanguinarine extract, metronidazole, quaternary ammonium compounds, such as cetylpyridinium chloride; cetylpyridium chloride clay complex, bis-guanides, such as chlorhexidine digluconate, hexetidine, octenidine, alexidine; and halogenated bisphenolic compounds, such as 2,2' methylenebis-(4-chloro-6-bromophenol).

Preferred levels of anti-microbial agents are from 0.01 to 5 wt% of the total composition, more preferably from 0.5 to 3 wt%, most preferably from 0.1 to 2 wt%.

The composition of the invention is used to clean the surfaces of the oral cavity and is known as an oral care composition.

Oral compositions of the invention are those which are suitable for brushing and/or rinsing the surfaces of the oral cavity.

In one preferred embodiment of the invention the composition is in the form of a dentifrice. The term "dentifrice" denotes an oral composition which is used to clean the surfaces of the oral cavity. Such a composition is not intentionally swallowed for purposes of systemic administration of therapeutic agents, but is applied to the oral cavity, used to treat the oral cavity and then expectorated. Typically, such a composition is used in conjunction with a cleaning implement such as a toothbrush, usually by applying it to the bristles of the toothbrush and then brushing the accessible surfaces of the oral cavity.

Preferably the dentifrice/toothpaste is in the form of an extrudable semi-solid such as a cream, paste or gel (or mixture thereof).

A composition according to the invention (such as a dentifrice/toothpaste) will generally contain further ingredients to enhance performance and/or consumer acceptability, in addition to the ingredients specified above.

Oral care tooth paste compositions as claimed comprise particulate abrasive materials such as silica and/or calcium carbonate usually in amounts between 3 and 60% by weight of the oral care composition.

The preferred abrasive silicas used in the present invention is a silica with a low refractive index. It may be used as the sole abrasive silica, or in conjunction with a low level of other abrasive silicas, e.g. those according to EP 236070. The low refractive index silicas, used as abrasives in the present invention are preferably silicas with an apparent refractive index (R.I.) in the range of 1.41 - 1.47, preferably 1.435 - 1.445, preferably having a weight mean particle size of between 5 and 15 mm, a BET (nitrogen) surface area of between 10 and 100 m²/g and an oil absorption of about 70 - 150 cm³/100 g, but abrasive silicas with a lower apparent refractive index may also be used. Typical examples of suitable low refractive index abrasive silicas (e.g. having an R.I. of between 1.435 and 1.445) are Tixosil 63 and 73 ex Rhone Poulenc; Sident 10 ex Degussa; Zeodent 113 ex Evonik; Zeodent 124 ex Evonik, Sorbosil AC 77 ex PQ Corporation (having an R.I. of approximately 1.440). The amount of these silicas in the composition generally ranges from 5-60% by weight, usually 5-20% by weight.

The oral care tooth paste composition preferably comprises an inorganic or a natural or synthetic thickener or gelling agent in proportions of about 0.10 to about 15% by weight depending on the material chosen. These proportions of thickeners in the dentifrice compositions of the present invention form an extrudable, shape-retaining product which can be squeezed from a tube onto a toothbrush and will not fall between the bristles of the brush but rather, will substantially maintain its shape thereon. Suitable thickeners or gelling agents useful in the practice of the present invention include inorganic thickening silicas such as amorphous silicas available from Huber Evonik under the trade designation Zeodent 165, Irish moss, iota-carrageenan, gum tragacanth, and polyvinylpyrrolidone.

Compositions according to the invention preferably comprise a polymeric deposition aid. Preferably the composition comprises acid anhydride polymers, particularly preferred are co-polymers of maleic anhydride with methyl vinylether, in which the anhydride moiety may be in a partially or fully hydrolysed or alcoholysed form. Preferred copolymers include Gantrez(R) polymers such as:
Gantrez S-95: molecular weight 216,000; free acid;
Gantrez S-96: molecular weight 700,000; free acid;
Gantrez S-97: molecular weight 1,500,000; free acid; and
Gantrez MS-955: molecular weight 1,060,000; calcium/sodium salt.

Particularly preferred co-polymers of maleic acid and methyl vinylether have a molecular weight of 1,000,000 or greater and an especially preferred material is Gantrez S-97.

Compositions according to the invention may comprise a tooth whitening agent. The whitening agent preferably comprises a green and/or a blue pigment. In the context of the present invention a pigment is generally understood to be a shade/material which is insoluble in the relevant medium, at the relevant temperature. This is in contrast to dyes which are soluble. In the context of this invention, the "relevant medium" is human saliva, the liquid medium in which the composition is used, at the temperature of the oral cavity during brushing of the teeth, i.e. up to 37 Degrees C. As a reasonable approximation, the relevant medium may be considered to be water and the relevant temperature to be 25 Degrees C.

Preferably the blue pigment is Pigment Blue 15, more preferably Pigment Blue 15:1, 15:2, 15:3, 15:4, 15:5 or 15:6, most preferably 15:1. A preferred pigment is blue pigment is Phthalocyanine Blue Pigment, Cl No. 74160, blue covarine.

The preferred Green pigment is Phthalocyanine Green, preferably Phthalocyanine Green CI-74260.

Preferably the total level of pigment in the composition is from 0.01 wt% to 3 wt, more preferably from 0.02 to 2 wt%.

If the composition is a toothpaste it may be a dual phase paste, with the whitening pigments present in one phase.

Compositions according to the invention may comprise oral care enzyme systems such as hydrogen peroxide producing enzyme systems (e.g. the oxidoreductase enzyme glucose oxidase), amyloglucosidase, dextranase and/or mutanase, (optionally in the presence of zinc ion providing compounds and/or 8- hydroxyquinoline derivatives), lactoperoxidase, lactoferrin, lysozyme and mixtures thereof.

In one embodiment a preferred class of oral care active for inclusion in the compositions of the invention includes agents for the remineralisation of teeth. The term "remineralisation" in the context of the present invention means the *in situ* generation of hydroxyapatite on teeth.

A specific example of a suitable agent for the remineralisation of teeth is a mixture of a calcium source and a phosphate source which, when delivered to the teeth results in the *in situ* generation of hydroxyapatite on teeth.

Illustrative examples of the types of calcium source that may be used in this context (hereinafter termed "remineralising calcium sources") include, for example, calcium phosphate, calcium gluconate, calcium oxide, calcium lactate, calcium glycerophosphate, calcium carbonate, calcium hydroxide, calcium sulphate, calcium carboxymethyl cellulose, calcium alginate, calcium salts of citric acid, calcium silicate and mixtures thereof. Preferably the remineralising calcium source is calcium silicate.

The amount of remineralising calcium source(s) (e.g. calcium silicate) in the composition of the invention typically ranges from 1 to 30%, preferably from 5 to 20% by total weight remineralising calcium source based on the total weight of the oral care composition.

Illustrative examples of the types of phosphate source that may be used in this context (hereinafter termed "remineralising phosphate sources") include, for example, monosodium dihydrogen phosphate, disodium hydrogen phosphate, sodium pyrophosphate, tetrasodium pyrophosphate, sodium tripolyphosphate, sodium hexametaphosphate, potassium dihydrogenphosphate, trisodium phosphate, tripotassium phosphate and mixtures thereof. Preferably the remineralising phosphate source is a mixture of trisodium phosphate and sodium dihydrogen phosphate.

The amount of remineralising phosphate source(s) (e.g. trisodium phosphate and sodium dihydrogen phosphate) in the composition of this invention typically ranges from 2 to 15%, preferably from 4 to 10% by total weight remineralising phosphate source based on the total weight of the oral care composition.

Mixtures of any of the above described materials may also be used.

The composition according the invention will comprise further ingredients which are common in the art, such as:
anti-inflammatory agents such as ibuprofen, flurbiprofen, aspirin, indomethacin etc.;
anti-caries agents such as sodium- and stannous fluoride, aminefluorides, sodium monofluorophosphate, sodium trimeta phosphate and casein;
plaque buffers such as urea, calcium lactate, calcium glycerophosphate and strontium polyacrylates;
vitamins such as Vitamins A, C and E;
plant extracts;
plant-derivable antioxidants such as flavonoid, catechin, polyphenol, and tannin compounds and mixtures thereof;
desensitising agents, e.g. potassium citrate, potassium chloride, potassium tartrate, potassium bicarbonate, potassium oxalate, potassium nitrate and strontium salts;
anti-calculus agents, e.g. alkali-metal pyrophosphates, hypophosphite-containing polymers, organic phosphonates and phosphocitrates etc.;
biomolecules, e.g. bacteriocins, antibodies, enzymes, etc.;
flavours, e.g. peppermint and spearmint oils;
proteinaceous materials such as collagen;
preservatives;
opacifying agents;
hyaluronic acid;
amino acids such as arginine;
colouring agents;
pH-adjusting agents;
sweetening agents;
pharmaceutically acceptable carriers, e.g. starch, sucrose, water or water/alcohol systems etc.; surfactants, such as anionic, nonionic, cationic and zwitterionic or amphoteric surfactants; Humectants such as glycerol, sorbitol, propyleneglycol, xylitol, lactitol etc.;
binders and thickeners such as sodium carboxymethyl-cellulose, hydroxyethyl cellulose (Natrosol^{®}), xanthan gum, gum arabic etc. as well as synthetic polymers such as polyacrylates and carboxyvinyl polymers such as Carbopol^{®};
polymeric compounds which can enhance the delivery of active ingredients such as antimicrobial agents can also be included;
buffers and salts to buffer the pH and ionic strength of the oral care composition; and
other optional ingredients that may be included are e.g. bleaching agents such as peroxy compounds e.g. potassium peroxydiphosphate, effervescing systems such as sodium bicarbonate/citric acid systems, colour change systems, and so on.

The invention will now be illustrated by the following non-limiting Examples

### Examples

*Neisseria elongata* was grown overnight, the resulting growth was collected. The cultures were washed twice using phosphate buffered saline (PBS) and resultant pellet resuspended in 10mls of PBS for use in the experiment.

Each lysine generation experiment consisted of 2ml CGVIII media (see appendix), 1ml 16% glucose and 1ml of either water, 100µM fumarate or aspartate or combinations at 0.5 parts of each of the individual solutions of fumarate, and aspartate. All solutions were adjusted to pH7 using sodium hydroxide. A 0.1ml aliquot of bacterial culture was added to the mixed solutions (table 1) Each combination was carried out in triplicate using 12 well plates.

### Experiment 1

**Table 1: Test solutions volumes (ml)**

| **Test** | **CGVIII** | **Glucose** | **Test agent** | **Bacteria** |
|---|---|---|---|---|
| Control (Media only) | 2 | 1 | 1 | 0.100 |
| Fumarate | 2 | 1 | 1 | 0.100 |
| Aspartate | 2 | 1 | 1 | 0.100 |
| Fumarate and Aspartate | 2 | 1 | 0.5/0.5 | 0.100 |

A 0.5 ml sample was collected for baseline lysine measurement before placing the solutions on to the orbital shaker for incubation at 37°C at 100rpm for 24 hours. A second 0.5ml sample was collected after 24 hours for lysine measurement. Solutions without bacteria were also generated as controls for background assessment.

After collection the sample was centrifuged at 4°C for 10 minutes at 15,000g, the supernatant was removed and frozen ready for analysis. Prior to analysis the samples were defrosted.

### Lysine analysis

Lysine present in the solutions was measured using a fluorogenic kit supplied by Abcam (ab273311) following the manufactures instructions. Modifications were made to the assay with the addition of a lysine standard curve rather than spiking with a lysine standard. Aliquots of 10ul from each sample, standard and background solutions were added to individual wells of a black microtitre plate followed by the addition of 50ul of lysine assay buffer. The reagents of the kit were added together to form the analysis reagent and 40ul added to each sample, standard and background solution. The plate was incubated at 25°C in the dark for 45 minutes prior to reading on a fluorescent plate reader at Ex/Em = 538/587nm. The data was exported into excel, where a calibration curve was generated, and the absorbance of the sample minus the background solutions were compared to give lysine concentrations.

### Results

Concentrations of lysine were determined for each test solution and the mean levels of lysine for the replicate solutions generated Table 1 shows the levels of lysine generated by fumarate and aspartate, alone and in combination (mean and standard deviation). Levels of lysine above that of the media only control were found for all solutions with boosted levels for the combination. Statistical analysis was carried out by comparing the means using T-Test.

**Table 2**

| Lysine concentrations at 0- and 24-hours following incubation of *Neisseria elongata* with fumarate and aspartate alone and in combination (mean and stdev) | | |
|---|---|---|
| | 0 hrs | 24 hrs |
| Control (media only) | 0.999 (0.047) | 12.729 (1.576) |
| Fumarate | 1.023 (0.040) | 14.747 (0.622) |
| Aspartate | 0.933 (0.109) | 15.639 (0.286) |
| Fumarate and Aspartate | 1.017 (0.039) | 23.833 (2.682) |

The results in Table 2 demonstrate that *Neisseria elongata* is capable of generating increased levels of lysine using fumarate and aspartate.

### Experiment 2

*Neisseria elongata* was grown overnight in brain heart infusion (BHI) broth at 37°C on an orbital shaker at 100rpm. The resulting growth was collected by centrifuging 2x30ml at 10000rpm for 5 minutes in 50ml tubes. The cultures were washed twice using phosphate buffered saline (PBS) and resultant pellet resuspended in 10mls of PBS for use in the experiment.

Each lysine generation experiment consisted of 2ml CGVIII media (see appendix), 1ml 16% glucose and 1ml either water, 100uM fumarate, aspartate or asparagine or combinations at 0.5 parts of each of the individual solutions, fumarate and aspartate of fumarate and asparagine. All solutions were adjusted to pH7 using sodium hydroxide and used to make a dilute toothpaste formulation using 0.3g of toothpaste plus 9.7g of each test solution. A 0.2ml aliquot of bacterial culture was added to the mixed solutions (table 1). Each combination was carried out in triplicate using 12 well plates.

**Table 3: Test solutions volumes (ml)**

| **Test** | **CGVIII** | **Glucose** | **Test agent** | **Bacteria** |
|---|---|---|---|---|
| Media only | 2 | 1 | 1 | 0.200 |
| Fumarate (Fum) + toothpaste | 2 | 1 | 1 | 0.200 |
| Aspartate (Asp) + toothpaste | 2 | 1 | 1 | 0.200 |
| Fum + Asp + toothpaste | 2 | 1 | 0.5/0.5 | 0.200 |

**Table 4 Toothpaste formulation**

| **Ingredient** | **Trade Name** | **wt%** |
|---|---|---|
| | | |
| Water and minors | | To 100 |
| Sorbitol (70%) | Neosorb 70/70 SB | 45.00 |
| Peg-32 | Pluracare PEG E1500 | 2.00 |
| Sodium Fluoride 98% | Sodium Fluoride 98% | 0.32 |
| Hydrated silica medium abrasivity | Zeodent 124 | 8.00 |
| PAS-Oral Care Grade | Empicol LZN, Ufarol TDG | 1.70 |
| Cellulose gum SCMC 9H | SCMC 9H | 0.80 |
| Sodium saccharin | Sodium saccharin | 0.20 |
| Titanium Dioxide | Titanium Dioxide-Anatase-Micro grade | 0.50 |
| Hydrating silica thickening med viscosity | Tixosil 43, Zeodent 165, Sorbisil TC15 | 8.50 |

Solutions were placed in an orbital shaker for incubation at 37°C and 100rpm for 24 hours. A 0.5ml sample was collected after 24 hours for lysine measurement. Solutions without bacteria were also generated as controls for background assessment.

After collection the samples was centrifuged at 4°C for 10 minutes at 15,000g, the supernatant was removed and frozen ready for analysis. Prior to analysis the samples were defrosted.

### Lysine analysis

Lysine present in the solutions was measured using a fluorogenic kit supplied by Abcam (ab273311) following the manufactures instructions. Modifications were made to the assay with the addition of a lysine standard curve rather than spiking with a lysine standard. Aliquots of 10ul from each sample, standard and background solutions were added to individual wells of a black microtitre plate followed by the addition of 50ul of lysine assay buffer. The reagents of the kit were added together to form the analysis reagent and 40ul added to each sample, standard and background solution. The plate was incubated at 25°C in the dark for 45 minutes prior to reading on a fluorescent plate reader at Ex/Em = 538/587nm. The data was exported into excel, where a calibration curve was generated, and the absorbance of the sample minus the background solutions were compared to give lysine concentrations.

### Results

Concentrations of lysine were determined for each test solution and the mean levels of lysine for the replicate solutions generated.

**Table 5**

| Lysine concentrations at 0- and 24-hours following incubation of *Neisseria elongata* with fumarate and aspartate alone and in combination (mean and stdev) in a 1:30 toothpaste formulation | |
|---|---|
| | **24 h** |
| Media only | 6.20854 (0.22704) |
| Fumarate (Fum) + toothpaste | 5.95336 (0.39795) |
| Aspartate (Asp) + toothpaste | 8.07153 (0.36127) |
| Fum + Asp + toothpaste | 9.21359 (1.31022) |

The results in Table 5 demonstrate that *Neisseria elongata* is capable of generating increased levels of lysine using fumarate and aspartate in a toothpaste

## Claims

1. An oral care tooth paste composition comprising:
i) particulate abrasive in which the particulate abrasive comprises silica and/or calcium carbonate.
ii) aspartic acid or salt thereof; and
iii) fumaric acid or sodium salt thereof

2. An oral care composition according to claim 1 which further comprises an anti-microbial agent.

3. An oral care composition according to claim 2 in which the anti-microbial agent comprises a source of fluoride in which the source of fluoride is preferably selected from the group consisting of sodium fluoride, stannous fluoride, sodium monofluorophosphate, zinc ammonium fluoride, tin ammonium fluoride, calcium fluoride, cobalt ammonium fluoride and mixtures thereof.

4. An oral care composition according to claim 3 in which the source of fluoride is sodium fluoride.

5. An oral care composition according to claim 2 in which the anti-microbial is a zinc salt preferably selected from the group consisting of zinc chloride, zinc acetate, zinc gluconate, zinc sulphate, zinc fluoride, zinc citrate, zinc lactate, zinc oxide, zinc monoglycerolate, zinc tartrate, zinc pyrophosphate and zinc maleate.

6. An oral care composition according to any preceding claim in which the level of aspartic acid or salt thereof is from 0.0001 to 5 wt% of the total composition, preferably from 0.001 to 3 wt%, most preferably from 0.01 to 1 wt%.

7. An oral care composition according to any preceding claim in which the level of fumaric acid or salt thereof is from 0.0001 to 5 wt% of the total composition, preferably from 0.001 to 3 wt%, most preferably from 0.01 to 1 wt%.

8. An oral care composition according to any preceding claim in which the weight ratio of aspartic acid or salt thereof to fumaric acid or salt thereof is from 1:2 to 2:1.

9. An oral care toothpaste composition comprising particulate abrasive, aspartic acid or salt thereof and fumaric acid or salt for use in preventing one or more of gingivitis, periodontitis, peri-implantitis, peri-implant mucositis, necrotizing gingivitis, gingival deterioration and detachment and mixtures thereof.

10. An oral care composition for use according to claim 9 in which the composition comprises at least one species of bacteria that has beneficial effects on oral health.

11. An oral care composition for use according to claim 10 in which the bacteria that has beneficial effects on oral health comprises *Neisseria elongate.*

## Patentansprüche

1. Mundpflege-Zahnpastazusammensetzung, umfassend:
i) partikelförmiges Schleifmittel, in welchem das partikelförmige Schleifmittel Siliciumdioxid und/oder Calciumcarbonat umfasst,
ii) Asparaginsäure oder ein Salz davon; und
iii) Fumarsäure oder ein Natriumsalz davon.

2. Mundpflegezusammensetzung nach Anspruch 1, die außerdem ein antimikrobielles Mittel umfasst.

3. Mundpflegezusammensetzung nach Anspruch 2, wobei das antimikrobielle Mittel eine Fluoridquelle umfasst, wobei die Fluoridquelle vorzugsweise aus der Gruppe ausgewählt ist, die aus Natriumfluorid, Zinnfluorid, Natriummonofluorphosphat, Zinkammoniumfluorid, Zinnammoniumfluorid, Calciumfluorid, Kobaltammoniumfluorid und Mischungen davon besteht.

4. Mundpflegezusammensetzung nach Anspruch 3, in der die Fluoridquelle Natriumfluorid ist.

5. Mundpflegezusammensetzung nach Anspruch 2, in welcher das antimikrobielle Mittel ein Zinksalz ist, vorzugsweise ausgewählt aus der Gruppe, bestehend aus Zinkchlorid, Zinkacetat, Zinkgluconat, Zinksulfat, Zinkfluorid, Zinkcitrat, Zinklactat, Zinkoxid, Zinkmonoglycerolat, Zinktartrat, Zinkpyrophosphat und Zinkmaleat.

6. Mundpflegezusammensetzung nach einem vorhergehenden Anspruch, in welcher der Gehalt an Asparaginsäure oder einem Salz davon 0,0001 bis 5 Gew.-% der Gesamtzusammensetzung beträgt, vorzugsweise 0,001 bis 3 Gew.-%, höchst bevorzugt 0,01 bis 1 Gew.-%.

7. Mundpflegezusammensetzung nach einem vorhergehenden Anspruch, in der der Gehalt an Fumarsäure oder einem Salz davon 0,0001 bis 5 Gew.-% der Gesamtzusammensetzung beträgt, vorzugsweise 0,001 bis 3 Gew.-%, höchst bevorzugt 0,01 bis 1 Gew.-%.

8. Mundpflegezusammensetzung nach einem vorhergehenden Anspruch, in welcher das Gewichtsverhältnis von Asparaginsäure oder einem Salz davon zu Fumarsäure oder einem Salz davon 1:2 bis 2:1 beträgt.

9. Mundpflege-Zahnpastazusammensetzung, umfassend partikelförmiges Schleifmittel, Asparaginsäure oder ein Salz davon und Fumarsäure oder ein Salz davon zur Verwendung bei der Vorbeugung einer oder mehrerer der folgenden Erkrankungen: Gingivitis, Parodontitis, Periimplantitis, periimplantäre Mukositis, nekrotisierende Gingivitis, Zahnfleischschädigung und -ablösung und Mischungen davon.

10. Mundpflegezusammensetzung zur Verwendung nach Anspruch 9, wobei die Zusammensetzung mindestens eine Bakterienart umfasst, die günstige Wirkungen auf die Mundgesundheit ausübt.

11. Mundpflegezusammensetzung zur Verwendung nach Anspruch 10, wobei die Bakterien, die günstige Wirkungen auf die Mundgesundheit ausüben, Neisseria elongate umfassen.

## Revendications

1. Composition de pâte dentifrice pour soins bucco-dentaires comprenant:
i) un abrasif particulaire dans laquelle l'abrasif particulaire comprend de la silice et/ou du carbonate de calcium;
ii) de l'acide aspartique ou un sel de celui-ci; et
iii) de l'acide fumarique ou un sel de sodium de celui-ci.

2. Composition pour soins bucco-dentaires selon la revendication 1, qui comprend en outre un agent antimicrobien.

3. Composition pour soins bucco-dentaires selon la revendication 2 dans laquelle l'agent antimicrobien comprend une source de fluorure dans laquelle la source de fluorure est choisie de préférence dans le groupe consistant en le fluorure de sodium, le fluorure stanneux, le monofluorophosphate de sodium, le fluorure de zinc et d'ammonium, le fluorure d'étain et d'ammonium, le fluorure de calcium, le fluorure de cobalt et d'ammonium et leurs mélanges.

4. Composition pour soins bucco-dentaires selon la revendication 3 dans laquelle la source de fluorure est le fluorure de sodium.

5. Composition pour soins bucco-dentaires selon la revendication 2 dans laquelle l'antimicrobien est un sel de zinc choisi de préférence dans le groupe consistant en le chlorure de zinc, l'acétate de zinc, le gluconate de zinc, le sulfate de zinc, le fluorure de zinc, le citrate de zinc, le lactate de zinc, l'oxyde de zinc, le monoglycérolate de zinc, le tartrate de zinc, le pyrophosphate de zinc et le maléate de zinc.

6. Composition pour soins bucco-dentaires selon l'une quelconque des revendications précédentes dans laquelle la teneur en acide aspartique ou en sel de celui-ci est de 0,0001 à 5 % en poids de la composition totale, de préférence de 0,001 à 3 % en poids, de manière particulièrement préférable de 0,01 à 1 % en poids.

7. Composition pour soins bucco-dentaires selon l'une quelconque des revendications précédentes dans laquelle la teneur en acide fumarique ou en sel de celui-ci est de 0,0001 à 5 % en poids de la composition totale, de préférence de 0,001 à 3 % en poids, de manière particulièrement préférable de 0,01 à 1 % en poids.

8. Composition pour soins bucco-dentaires selon l'une quelconque des revendications précédentes dans laquelle le rapport pondéral de l'acide aspartique ou sel de celui-ci à l'acide fumarique ou sel de celui-ci est de 1:2 à 2:1.

9. Composition de pâte dentifrice pour soins bucco-dentaires comprenant un abrasif particulaire, de l'acide aspartique ou un sel de celui-ci et de l'acide fumarique ou un sel de celui-ci destinée à être utilisée dans la prévention d'une ou plusieurs parmi la gingivite, la parodontite, la péri-implantite, la mucite péri-implantaire, la gingivite nécrosante, la détérioration et le décollement gingivaux, et leurs mélanges.

10. Composition pour soins bucco-dentaires destinée à être utilisée selon la revendication 9 dans laquelle la composition comprend au moins une espèce de bactérie qui a des effets bénéfiques pour la santé bucco-dentaire.

11. Composition pour soins bucco-dentaires destinée à être utilisée selon la revendication 10 dans laquelle la bactérie qui a des effets bénéfiques sur la santé bucco-dentaire est *Neisseria elongate.*
